(19) Europäisches Patentamt
European Patent Office
Office européen des brevets

(11) Numéro de publication: **0 018 259**
**B1**

(12) **FASCICULE DE BREVET EUROPEEN**

(45) Date de publication du fascicule du brevet: 08.12.82

(51) Int. Cl.³: **C 07 D 317/36, C 22 B 3/00, C 08 G 65/32**

(21) Numéro de dépôt: **80400445.5**

(22) Date de dépôt: **03.04.80**

(54) **Nouveaux carbonates porteurs de groupes carbonates cycliques, leur procédé de fabrication et leur application à l'extraction de valeurs métalliques à partir de solutions aqueuses.**

(30) Priorité: **13.04.79 FR 7909402**

(43) Date de publication de la demande: **29.10.80 Bulletin 80/22**

(45) Mention de la délivrance du brevet: **08.12.82 Bulletin 82/49**

(84) Etats contractants désignés: **BE CH DE FR GB IT LI NL SE**

(56) Documents cités:
**US-A-3 542 841**
**US-A-3 912 801**

(73) Titulaire: **SOCIETE NATIONALE DES POUDRES ET EXPLOSIFS, 12, quai Henri IV, F-75181 Paris, Cedex 04 (FR)**

(72) Inventeur: **Burgard, Michel, 34, rue de Fréland, F-67100 Strasbourg (FR)**
Inventeur: **Rollat, Alain, Boite Postale, 1017, Alger (DZ)**
Inventeur: **Piteau, Marc, 38, avenue de Ballancourt, F-91710 Itteville (FR)**
Inventeur: **Senet, Jean-Pierre, 79, rue de la Gare Herbeauvilliers-Buthiers, F-77760 La Chapelle La Reine (FR)**

**EP 0 018 259 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

# Nouveaux carbonates porteurs de groupes carbonates cycliques, leur procédé de fabrication et leur application à l'extraction de valeurs métalliques à partir de solutions aqueuses

La présente invention se rapporte à des composés porteurs de groupes carbonates cycliques, aux procédés de fabrication de tels composés et à leur application en hydrométallurgie.

Il est connu par le brevet américain 2 446 145 de préparer le chloroformiate de dioxycarbonyl-2,3 propyle par action du phosgène sur le glycérol. Il est également connu, par le même document, d'obtenir le carbamate et des uréthannes correspondants par action de ce chloroformiate sur l'ammoniac ou des amines.

Par ailleurs on connait l'intérêt de certaines substances organiques qui ont la faculté d'extraire tout ou partie des ions métalliques contenue dans des solutions aqueuses. De telles solutions de lixiviation de minerais, de solutions résultant de l'attaque de vieux métaux recyclés, ou, aussi bien, d'effluents industriels nuisibles pour l'environnement.

Fondementalement ces substances organiques, qu'on dénomme agents extractants, doivent être capables non seulement d'extraire plus ou moins sélectivement des ions métalliques de solutions aqueuses mais encore de les restituer à une nouvelle solution aqueuse pure et plus concentrée qu'on traite ensuite d'une manière connue en soi.

Toutefois, pour qu'on puisse retirer des agents extractants tous les avantages propres aux techniques hydrométallurgiques (souplesse, otention de métaux d'une grande pureté, automation, fonctionnement en continu et surtout, mise en valeur des minerais ou des déchets), il faut encore que lesdits agents extractants possèdent de nombreuses autres qualités:

— faible solubilité dans les phases aqueuses (causes de perte)
— faible volatilité (également cause de perte)
— bonne solubilité dans des milieux organiques peu coûteux
— bonne reversibilité
— vitesse d'extraction élevée
— sélectivité (dans le cas de solutions complexes)
— capacité d'extraction élevée
— coefficient d'extraction élevé
— bonne tenue aux recyclages,
— toxicité faible ou nulle
— caractère corrosif faible ou nul.

Dans un article paru dans la revue HYDROMETALLURGY en 1976, volume 1, pages 207–240 et intitulé »Solvent Extraction of Non ferrous metals«, D. S. FLETT et D. R. SPINK font le point sur les principaux agents extractants connus, avec, pour chacun, leur spectre d'activité et leurs conditions d'utilisation. On distingue ainsi les agents extractants solvatants, acides, chélatants et ioniques. Parmi les agents extractants solvatants on compte des phosphates tels que le tributylphosphate (TBP), des cétones telles que la méthyl isobutyl cétone (ou MIBK) ou l'isophorone (brevet US-A-4 008 308), des éthers tel l'éther dibutylique du glycol (BUTEX®). Parmi les agents extractants acides on compte des acides naphténiques et versatiques et, surtout, l'acide diéthylhexyl-2 phosphorique (ou D2EHPA) (brevet US-A-3 989 607 et FR-A-2 342 346 et FR-A-2 367 832, par exemple). Parmi les agents extractants chélatants on compte essentiellement des $\alpha$-hydroxyoximes (connus commercialement sous le nom de LIX) qui sont décrits dans US-A-3 224 873 et US-A-3 449 066. Enfin, parmi les agents extractants ioniques on compte principalement des amines et des sels d'ammoniums quaternaires, comme ceux décrits dans FR-A-1 266 363.

Par zilleurs, US-A-3 072 613 déout des di (carbonyl dioxy-2,3 propyle carbonates) d'alcanes bisphénoliques qui conduisent, après réactù sur des diamines primaires, à des polymères utiles comme adhérifs, vernis, filmogéues et Fibres. Ces apllications n'ontaucius rapport avec l'hydrométallurgie. L'invention concerne quant à elle des composés chimiques nouveaux qui, dans leur application comme agents extractants, ne s'apparentent à aucun type connu et par la même ont un spectre d'application particulier, propre à permettre les résolutions de problèmes généraux ou spécifiques en matière d'extraction.

Les carbonates porteurs de groupes carbonates cycliques selon l'invention ont pour formule générale

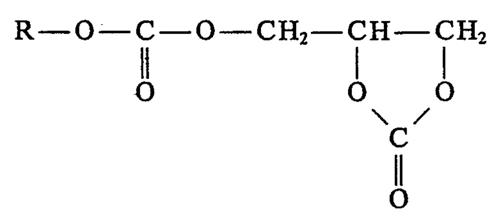

(A)

où R est un groupe alkyle linéaire ou ramifié comportant de 1 à 20 atomes de carbone, éventuellement substitué par un à trois groupes

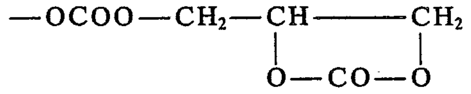

- un groupe alicyclique comportant de 4 à 20 atomes de carbone, ou un groupe aralkyle comportant de 7 à 20 atomes de carbone, éventuellement substitués par un à trois groupes

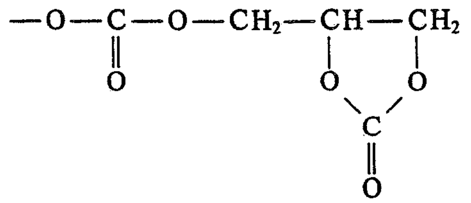

- un groupe carboxylate $-COOR''$ où R″ comporte de 1 à 19 atomes de carbone, éventuellement substitué par un groupe

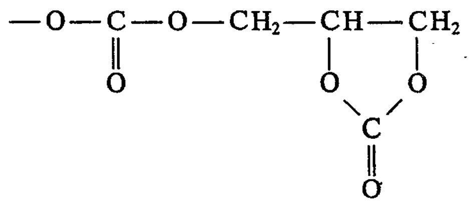

- un groupe

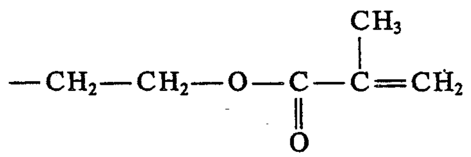

- un polyoxyéthylène de formule:

$$R'-(O-CH_2-CH_2)_n-$$

où n est compris entre 1 et 40, où R′ est un groupe hydrocarbyle comprenant de 1 à 10 atomes de carbone et porte éventuellement une ou deux chaines

$$-(O-CH_2-CH_2)_{n'}-,$$

où n′ est compris entre 1 et 40, terminées par un groupe

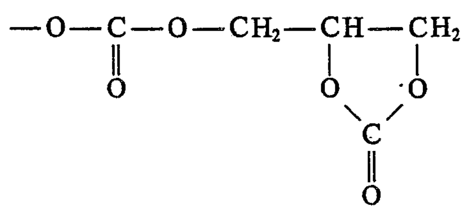

- un polyoxypropylène de formule

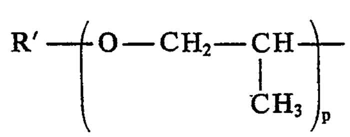

où p est compris entre 1 et 40 et où R′ est un groupe hydrocarbyle comprenant de 1 à 10 atomes de carbone et porte éventuellement une ou deux chaines

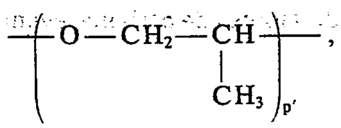

où p' est compris entre 1 et 40, terminées par un groupe

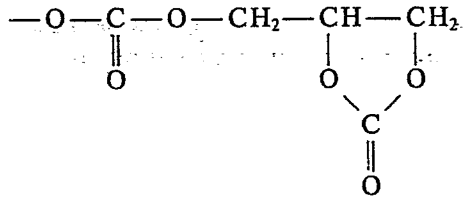

- un groupe aryle comportant de 6 à 20 atomes de carbone,
- un polyester de formule

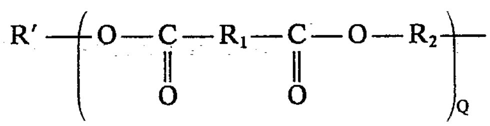

où Q est compris entre 1 et 20, où $R_1$ et $R_2$ sont identiques ou différents et sont une chaine polyméthylène ayant de 1 à 8 atomes de carbone ou une chaine polyéther ayant de 1 à 8 atomes de carbone et où R' est un groupe hydrocarbyle comprenant de 1 à 10 atomes de carbone, ou bien,

- un polycarbonate aliphatique

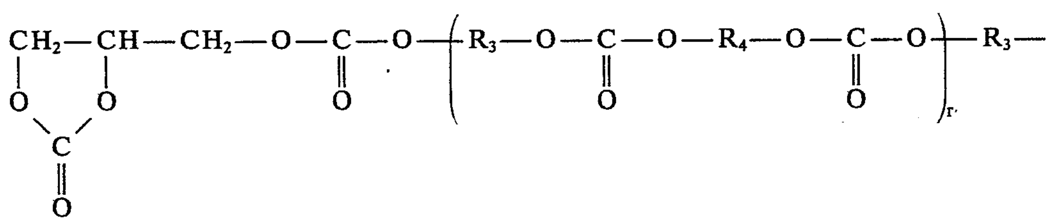

où r est compris entre 1 et 20 et où $R_3$ et $R_4$ sont identiques ou différents ou tantôt identiques, tantôt différents et sont des groupes polyméthylène comportant de 2 à 8 atomes de carbone ou des groupes polyoxyéthylène

$$-(CH_2-CH_2-O)_t-CH_2-CH_2-$$

où t est égal à 1, 2, 3, 4 ou 5.

L'invention concerne également un procédé de fabrication des nouveaux produits qui viennent d'être décrits.

Le procédé selon l'invention consiste à faire agir le chloroformiate de carbonyl dioxy-2,3 propyle sur un alcool de formule générale ROH où R a l'une des significations précédemment exposées des groupes OH remplaçant les groupes $CO_3-CH_2-(CH-CH_2)CO_3$ éventuels à une température comprise entre −10 et +20° C et en présence d'un accepteur d'acide.

Conformément à l'invention, l'accepteur d'acide peut être une base minérale ou organique. Comme base minérale on peut utiliser la soude, la potasse, le carbonate de sodium, le carbonate de potassium ainsi que le bicarbonate de sordium. Toutefois, on préfère utiliser des bases organiques et, parmi celles-ci, les amines tertiaires aliphatiques ou aromatiques sont préférées. Plus particulièrement on préfère la pyridine et la triéthylamine bien que d'autres amines puissent être utilisées sans qu'on en tire d'avantages ou d'inconvénients particuliers. Dans le cas où R est un groupe aryle et ROH un composé phénolique, on utilise de préférence la soude comme accepteur, le chloroformiate agissant alors sur le phénate RONa.

Contrairement à ce que la sensibilité des fonctions carbonates et carbonates cycliques vis à vis des bases laissait craindre, il ne se produit pas, dans les conditions précises exposées plus haut, de décarbonatation ni de transcarbonatation du chloroformiate utilisé ou des produits formés, au fur et à mesure de l'avancement de la réaction. Par ailleurs, le rendement de la transformation de l'alcool en son carbonate est toujours élevé puisque supérieur généralement à 80% et, le plus souvent, supérieur à 85%. De plus, d'une manière surprenante, le rendement reste également bon même lorsqu'un polyol est utilisé comme réactif de départ.

Un solvant est en principe utilisé comme milieu réactionnel encore que le produit formé constitue lui-même un excellent solvant pour les réactifs, de sorte que le procédé n'exige donc pas forcément la

présence d'un solvant. Comme solvants convenables on peut citer les solvants organiques des alcools et polyols concernés et du chloroformiate de carbonyl dioxy-2,3 propyle et qui sont inertes vis à vis de ces réactifs ainsi que vis à vis de l'accepteur d'acide. On peut citer par exemple les hydrocarbures aliphatiques et aromatiques, les hydrocarbures halogénés, les éther, les cétones, les esters tels que l'hexane, le benzène, le toluène, le xylène, le tétrachlorure ce darbone, le chloroforme, le dichlorométhane, le tétrahydrofuranne, les glymes, l'acétone, la cyclohexanone, l'acétate d'éthyle.

Les proportions de réactifs à utiliser sont celles correspondant à la stoechiometrie de la réaction, c'est à dire qu'on fait réagir au moins une mole de chloroformiate par mole d'hydroxyle porté par l'alcool, en présence d'une quantité équimoléculaire d'accepteur d'acide. De préférence, on utilisera un léger excès de chloroformiate, de préférence de 5 à 15%, molaire et un excès correspondant d'accepteur d'acide.

Une fois la réaction terminée ce qui nécessite de 0,5 à 5 heures, on lave avantageusement le milieu réactionnel à l'eau acidulée puis à l'eau pure, après quoi on sèche ledit mélange sur un sel déshydratant tel qu'un sulfate alcalin ou alcalino-terreux et on évapore le solvant: le produit obtenu ne nécessite, dans la plupart des cas, aucun traitement de purification.

L'invention concerne également un procédé de séparation de valeurs métalliques à partir d'une solution aqueuse les contenant par extraction liquide — liquide caractérisé en ce que (1) on met lesdites solutions aqueuses en contact avec une phase organique comprenant un composé de formule générale:

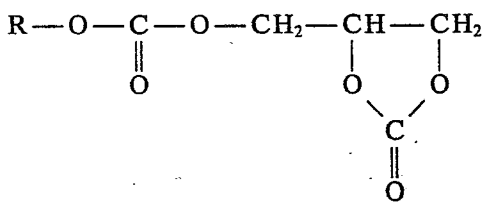

(A)

éventuellement en solution dans un solvant organique sensiblement non miscible à l'eau, si bien qu'une partie au moins desdites valeurs métalliques ext extraite dans la phase organique (2) on sépare la phas phase organique chargée contenant les valeurs métalliques extraites de la soultion aqueuse sous forme d'un complexe avec le composé de formule générale (A) et (3) on récupère les valeurs métalliques de la phase organique par mise en contact de cette dernière avec un milieu de strippage aqueux.

Les valeurs métalliques particulièrement intéressées par l'invention sont notamment les ions métalliques: Zn (II), Cd (II), Hg (II), Fe (III), Co(II), Cu(I), Rh (III), Cu (II), Pd (II), Pt (II), Mo (V), V (IV), Pb (II), Ag (I), Au (III), Ga (III) ainsi que Na (I), K (I), Hg (I), R (IV), U (VI) et les lanthanides aux degrés d'oxydation (II), (III) ou (IV).

Des problèmes classiques posés mais qui peuvent être résolus grâce à l'utilisation des composés de formule générale (A) sont par exemple l'extraction de cobalt en présence de nickel, l'extraction de zinc en présence de nickel, l'extraction de molybdène en présence d'uranium et de strontium et l'extraction de l'or en présence de métaux de la mine du platine.

Les contre-ions qu'on peut trouver dans les solutions aqueuses concernées par l'invention sont essentiellement $NO_3^-$, $Cl^-$, $ClO_4^-$, $So_4^{2-}$, et $SCN^-$, ce dernier étant préféré dans la plupart des cas sauf dans le cas de l'or où un milieu $Cl^-/NO_3^-$ est particulièrement convenable.

Les solvants organiques sensiblement non miscibles à l'eau associés de préférence aux composés selon l'invention au sein de la phase organique d'extraction sont essentiellement les hydrocarbures aliphatiques chlorés tels que, par exemple, les kérosènes, le benzène, le touène, le xylène, le dichloréthane, le chloroforme et d'une manière générale, les solvants organiques ayant un point éclair supérieur à 65° C.

La gamme des pH utilisables dans la phase aqueuse chargée des valeurs métalliques à extraire et dans le milieu de strippage est comprise entre — 1 et 12, de préférence entre 0 et 10, ce qui n'est pas différent des conditions de pH ordinairement rencontrées dans les procédés antérieurs. Il est précisé qu'on doit entre par strippage le transfert d'au moins une partie du ou des métaux présents dans la phase organique dans un milieu aqueux dit »milieu de strippage« lequel peut être soumis de façon connue en soi à un traitement ultérieur de récupération desdits métaux, ainsi obtenus à l'état très pur. Naturellement chaque solution aquese contenant du métal de départ est ributaire de ses propres conditions optimales, ainsi que les spécilaistes de la technique le comprendront parfaitement bien.

Bien que les composés selon l'invention puissent constituer à eux seuls la phase organique d'extraction, il est généralement suffisant d'en utiliser des solutions diluées dans un solvant organique de type très général défini plus haut. On peut toutefois utiliser des solutions concentrées de l'ordre de 50 à 75% en poids afin de faciliter le transport et la manipulation et procéder à des dilutions subséquentes éventuelles au moment de l'emploi pour obtenir des solutions contenant au moins 1%, de préférence au moins 5 à 10%, en poids d'agent extractant selon l'invention, par rapport au solvant.

Parmi les composés de formule générale (A) selon l'invention certains sont particulièrement préférés dans leur application à l'extraction liquide — liquide des valeurs métalliques.

En vue de l'extraction de l'or des solutions aqueuses le contenant, on utilise avantageusement notamment le carbonate de carbonyl dioxy-2,3 propyle et de méthyle, de n-propyle, de n-butyle, de n-pentyle, de n-hexyle, de n-heptyle, de n-octyle et leurs homologues linéaires supérieurs jusqu'à octa décyle ainsi que le carbonate de bis(carbonyl dioxy-2,3 propyle) et de glycol ou d'hexane diol-1,6, le carbonate de carbonyl dioxy-2,3 propyle et de benzyle, le carbonate de carbonyle dioxy-2,3 proyple et de phényle, le carbonate de bis(carbonyl dioxy-2,3 propyle) et de tri, tétra, penta, hexa ou hepta éthylène glycol, le carbonate de tri(carbonyl dioxy-2,3 propyle) et triméthylol propane, les carbonates de bis(carbonyl dioxy-2,3 propyle) et de polycarbonate de diéthylène glycol de masse inférieure à 3000, et les carbonates de bis(carbonyl dioxy-2,3 propyle) et de polycarbonate de butane diol de masse inférieure à 2000 ainsi que les carbonates de bis(carbonyl dioxy-2,3 propyle) et de polycarbonates séquencés ou non d'hexane diol et de butane diol.

En vue d'extraction du zinc, du molybdène et du cobalt et des métaux autres que l'or et les métaux de la mine du platine on utilise avantageusement les polyoxyéthylènes de formule

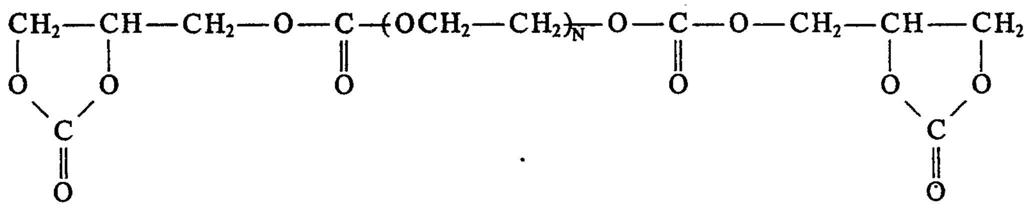

où N est égal ou supérieur à 2 et dont la masse molaire ne dépasse pas, de préférence, 5000 et, dans une moindre mesure, les carbonates de bis(carbonyl dioxy-2,3 propyle) et de diol et les carbonates de tri(carbonyl dioxy-2,3 propyle) et de triol, obtenus par condensation d'oxyde de propylène sur des diols ou des triols aliphatiques comprenant de 2 à 8 atomes de carbone, et de masse globale inférieure de préférence à 3000.

On appréciera que les composés selon l'invention outre leur capacité d'extraire des valeurs métalliques de solutions auqueuses les contenant, ont des propriétés physiques et chimiques les rendant extrêmement attrayants. Ils sont pour la plupart en effet peu ou pas solubles dans l'eau et réciproquement. Ils sont au contraire bien solubles dans les solvants couramment utilisés en hydrométallurgie et possèdent une masse volumique généralement de l'ordre de 1,4 t/m$^3$, ce que est nettement supérieur à celle des solutions aqueuses chargées en valerrs métalliques. Leur volatilité et leur toxicité est très faible. En revanche ils supportent très bien, grâce à la présence des fonctions carbonates, les contraintes mécaniques élevées auxquelles sont soumis les agents d'extractions d'où une aptitude très satisfaisante au recyclage. Enfin, ni les composés en eux mêmes, ni les produits qui pourraient résulter de leur décomposition accidentelle, ne peuvent exercer une action corrosive sur les appareillages industriels.

Ces qualités donnent un avantage considérable aux composés selon l'invention notamment vis à vis des carbonates d'éthylène et de propylène, connus comme extractants par le brevet US-A-3 912 801 mais industriellement difficiles à mettre valablement en oeuvre du fait de leur solubilité dans l'eau, de leur faible poids moléculaire et leur relative instabilité dans des conditions basiques.

D'autres avantages des produits et du procédé selon l'invention apparaitront dans les exemples suivants qui ne sont donnés qu'à titre d'illustration de l'invention et ne sauraient par conséquent limiter cette dernière.

## Exemple 1

### Préparation du chloroformiate de carbonyl dioxy-2,3 propyle

Dans un réacteur muni d'un réfrigérant à reflux à 40° C, d'un thermomètre, d'un agitateur et d'une ampoule de coulée, on introduit 500 ml de phosgène liquide.

On coule lentement, en 3 heures environ, 276 g (3 moles) de glycérol en maintenant la température entre 0 et +5° C.

On agite 1 heure à 0° C puis laisse revenir en 2 heures à température ambiante.

On fait passer un courant d'azote jusqu'à élimination complète du phosgène en excès, ajoute 400 ml de dichlorométhane et lave 3 fois avec 500 ml d'eau glacée.

La phase organique est recueillie et séchée sur sulfate de sodium. Après évaporation du solvant sous pression réduite et à 30° C, on obtient 348 g de produit soit un rendement de 64% par rapport au glycérol.

Analyse:

- taux de chlore hydrolysable: 20,2% (théorie 19,56%)
- Spectre infra-rouge bandes d'absorption:
  - $\nu$ >C=O (chloroformiate) à 1780 $cm^{-1}$ et
  - $\nu$ >C=O (carbonate cyclique) à 1800 $cm^{-1}$

Exemple 2

Dans un réacteur muni d'un réfrigérant à reflux, d'un thermomètre d'un agitateur et d'une ampoule de coulée, on introduit

- 346 g (1,9 mole) de chloroformiate préparé précédemment
- 247 g de n-octanol (1,9 mole)
- 600 ml de dichlorométhane

On coule en 1 heure environ 152 g de pyridine (2,2 moles) en maintenant la température entre −5 et 5° C, (soit un excès molaire de 15%).

On agite 1 heure à 0° C puis laisse remonter à température ambiante en 2 heures environ.

Le chlorhydrate de pyridine est éliminé par filtration et la phase organique lavée avec deux fois 500 ml d'eau.

Après séchage sur sulfate de magnésium et élimination du solvant par évaporation sous pression réduite, le produit est étêté à 100° C sous 1 mm Hg (133 Pa) pendant 30 mn.

On obtient ainsi 460 g d'une huile incolore qui cristallise à température ambiante. Le rendement est de 88% par rapport au chloroformiate.

Analyses:

- Spectre infra-rouge: bandes d'absorption
  - $\nu$ C=O (carbonate linéaire) à 1740 $cm^{-1}$ et
  - $\nu$ C=O (carbonate cyclique) à 1800 $cm^{-1}$
- Spectre RMN en accord avec la formule.

Il est à noter que les carbonates d'alkyle plus légers ou plus lourds peuvent être synthétisés avec la même facilité, dans les mêmes conditions et avec un succès comparable à ce qui vient d'être rapporté.

Exemple 3

Synthèse du tri(carbonate de carbonyle dioxy-2,3 propyle) de triméthylolpropane

Dans un réacteur de 2 litres on introduit 93,8 g (0,7 mole) de triméthylolpropane, 400 $cm^3$ d'acétone et 183 g (2,31 moles) de pyridine. La température étant maintenue vers 0° C, on additionne une solution de 417 g (2,31 moles) de chloroformiate de carbonyle dioxy-2,3 propyle dans 400 $cm^3$ d'acétone. Après deux heures d'agitation à température ambiante le amélange réactionnel est filtré, le filtrat est concentré sous pression réduite, repris par 1,5 litre de dichlorométhane lavé à l'eau acidulée puis à l'eau pure. La solution organique est séchée, filtrée et concentrée sous pression réduite. On obtient ainsi 347 g (rendement 88%) de tricarbonyle dioxy-2,3 propyle carbonate de triméthylolpropane.

- Point de fusion: 120° C
- Taux résiduel d'OH: 0%
- Spectre infra-rouge:

$$\nu\ \underset{\overset{\|}{O}}{C}\ \text{(carbonate linéaire)}: 1750\ cm^{-1}$$

$$\nu\ \underset{\overset{\|}{O}}{C}\ \text{(carbonate cyclique)}: 1800\ cm^{-1}$$

Ce spectre est tout à fait conforme à la théorie.

## Exemple 4

Synthèse du di(carbonyl dioxy-2,3 propyle carbonate) de tétraéthylène glycol

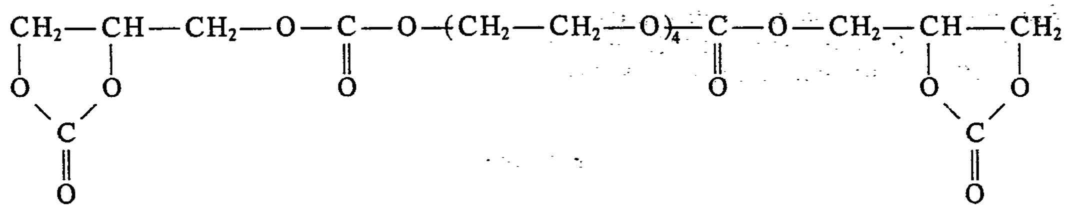

Dans un réacteur de 20 l en verre, on introduit 2,888 kg (16 moles) de chloroformiate de carbonyl dioxy-2,3 propyle et 5 l de dichlorométhane anhydre.

La température étant maintenue entre −4 et +2° C, on coule lentement sous agitation un mélange de:

- 1,552 kg (8 moles) de tétraéthylène glycol
- 1,430 kg de pyridine (18,1 moles) (soit un excès de 13% par rapport à la stochiométrie)
- 1 l de dichlorométhane anhydre.

Après la coulée qui dure 1 h 45 mn, on réchauffe le milieu réactionnel entre +15 et +18° C et on maintient cette température pendant une heure.

On ajoute 7 l d'eau salée et sépare la phase organique. Cette dernière est lavée trois fois avec un mélange de 7 litres d'eau salée et 500 ml d'acide chlorhydrique concentré, deux fois avec 7 litres salée et une fois avec 7 litres d'eau déminéralisée.

La phase organique est séchée sur sulfate de sodium anhydre et le solvant est éliminé par évaporation sous pression réduite à 50° C. On recueille ainsi 3,225 kg (rendement 83,6%) d'un produit très visqueux dont les principales caractéristiques sont les suivants:

- teneur en eau: 0,040%
- teneur en fonctions OH résiduelles $\leq 10^{-2}$ eq/kg

Dicarbonate de carbonyl dioxy-2,3 propyle et de tétraéthylène

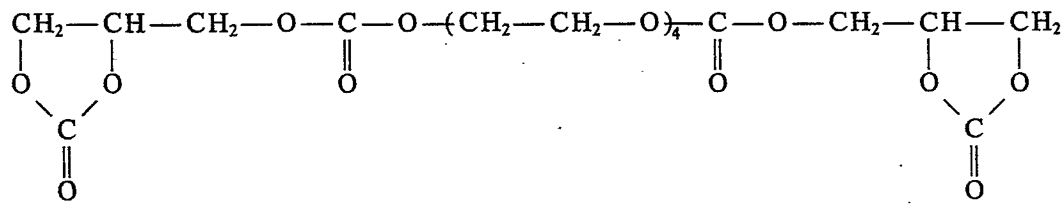

Liquide visqueux, légèrement ambré, possédant une faible odeur:

- Fusion: −14° C (point de transition)
- Tension de vapeur
  à 20° C: 5 mm/Hg (666 Pa)
  à 100° C: 22 mm/Hg (2933 Pa)
- Densité $d^{25}$: 1,41
- Viscosité à 25° C: 60 000 CP
- Indice de réfraction 25° C $N_D^{25}$: 1,4750
- Solubilité: insoluble dans l'eau, bonne solubilité dans les solvants organiques (60 g dans 100 g de $CH_2Cl_2$ à 20° C).

## Exemple 5

Synthèse du di(carbonyl dioxy-2,3 propyl carbonate) de polyoxyéthylène glycol

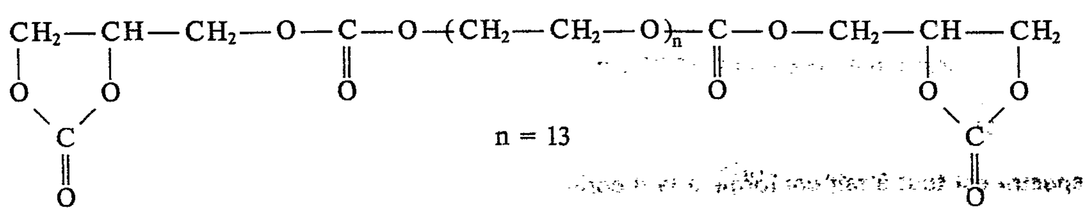

n = 13

Dans un réacteur de 2 litres, on introduit 300 g (0,5 mole) de polyéthylène glycol (Mn = 600), 300 ml de dichlorométhane anhydre et 87 g (1,1 mole) de pyridine.

La température étant maintenue entre 0 et +5° C, on ajoute goutte à goutte, sous agitation, 198,5 g (1,1 mole) de chloroformiate de carbonyl dioxy-2,3 propyle en solution dans 300 ml de dichlorométhane. Après deux heures d'agitation à température ambiante, le mélange réactionnel est filtré et le filtrat obtenue est lavé avec une solution aqueuse d'acide chlorhydrique puis à l'eau distillée jusqu'à obtention de la neutralité des eaux de lavage.

La phase organique est séchée sur sulfate de magnésium et le solvant est éliminé par évaporation sous pression réduite.

On recueille ainsi 366,7 g (rendement 82,6%) de produit dont le spectre infra-rouge est correct.

- Teneur en fonctions OH résiduelles: 0,01 eq/kg
- Teneur en chlore total ≤400 ppm
- Mn = 890.

Soluble: acétone, chloroforme, éther, toluène, benzène.

Exemple 6

Synthèse du di(carbonyl dioxy-2,3 propyle carbonate) de polycarbonate de diéthylène glycol

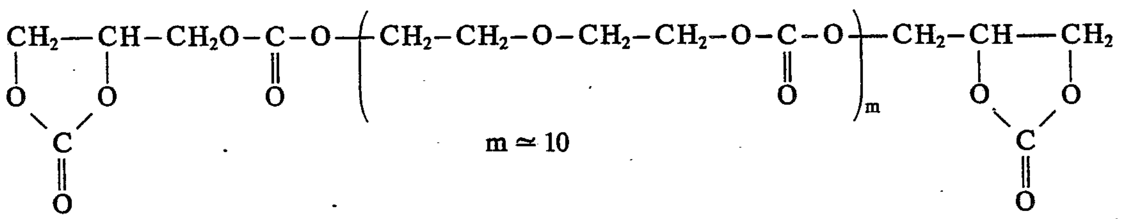

m ≃ 10

Le mode opératoire utilisé est strictement identique au précédent (exemple 5) avec:

- 410 g (0,33 mole) de polycarbonate de diéthylène glycol
- 58 g de pyridine (0,73 mole) (soit un excès de 10% par rapport à la stoechiométrie)
- 600 ml de dichlorométhane anhydre
- 132 g de chloroformiate de carbonyl dioxy-2,3 propyle (0,73 mole).

On recueille ainsi 435 g de produit dont le spectre infra-rouge est conforme à la formule donnée ci-dessus et dont la masse molaire en nombre est environ 1500, la teneur en fonctions OH est inférieur ou égale à 0,02 eq/kg et le taux de chlore total est de 0,06%.

Exemple 7

Synthèse du carbonate de carbonyl dioxy-2,3 propyle et de méthacrylate d'éthyle

Dans un réacteur de 250 ml et à l'abri de l'humidité on place 11,4 g (0,1 mole) d'hydroxy-2 éthylméthacrylate, 9 g (0,11 mole) de pyridine et 50 ml de chloroformiate de carbonyl dioxy-2,3 propyle dans 30 cm³ de chlorure de méthylène. On laisse encore agiter une heure à température ambiante puis on additionne 50 cm³ d'eau acidulée. La phase organique est décantée, séchée et évaporée sous pression réduite. On obtient de cette manière 23 g (rendement: 84%) de carbonate mixte de carbonyl dioxy-2,3 propyle et de méthacrylate d'éthyle.

- Spectre infra-rouge:
  - ν C=O (carbonate cyclique): 1830 $cm^{-1}$
  - ν C=O (carbonate linéaire): 1755 $cm^{-1}$
  - ν C=O (ester): 1720 $cm^{-1}$
  - ν C=C: 1630 $cm^{-1}$
- Spectre de résonance magnétique nucléaire:

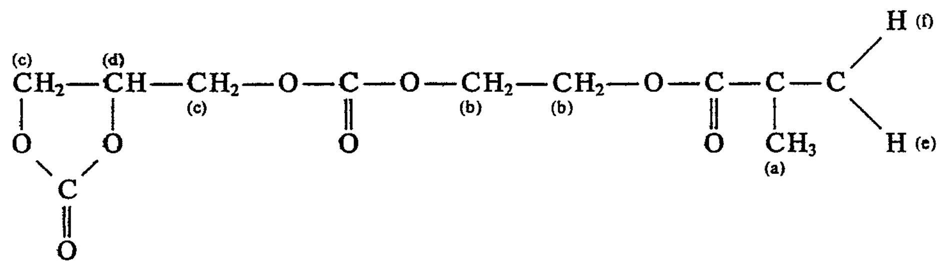

(a) massif à 1,98 ppm 3 H
(b) singulet à 4,38 ppm 9 H
(c) massif complexe de 4,1 à 4,75 ppm 9 H
(d) massif de 4,7 à 5,1 ppm 9 H
(e) massif à 5,6 ppm 1 H
(f) massif à 6,13 ppm 1 H

Exemples 8 à 13

Extraction de l'or

On a préparé une solution d'or reproduisant des conditions habituellement rencontrées dans les solutions de lixiviation des minerais aurifères. Cette solution renfermait 100 ppm d'Au sous forme de Na Au $Cl_4$ avec NaCl (2 moles) en sel de fond. Son pH était égal à 1,7.

On a mélangé un volume de cette solution aqueuse avec un volume d'une solution molaire de composés selon l'invention dans divers solvants. Après agitation des deux phases, on a mesuré le pourcentage d'or extrait de la phase aqueuse et présent dans la phase organique. Les résultats sont rassemblés dans le tableau I suivant

Tableau 1

| Ex. | Extractant | Solvant | % or extrait |
|---|---|---|---|
| 8 | Composé de l'exemple 2 | Benzène | 41 |
| 9 | do | Chloroforme | 40 |
| 10 | Composé de l'exemple 4[1]) | Dichloroéthane | 100 |
| 11 | do | Chloroforme | 100 |
| 12 | Composé de l'exemple 5 | Chloroforme | 100 |

[1]) mais avec 3 ponts éthers au lieu de 4.

A titre de comparaison, (exemple 13) la méthyl isobutyl cétone permet également d'atteindre une extraction totale de l'or, mais à condition d'être utilisée pure, non diluée dans un solvant. Par ailleurs, il s'agit d'un acomposé relaltivement volatil, légèrement soluble dans l'eau et de densité inférieure à celle de l'eau.

Exemples 14 à 17

Extraction de l'or en présence de platine

On a essayé d'extraire sélectivement l'or d'une phase aqueuse contenant à la fois de l'or (sous forme de Na $AuCl_4$ comme à l'exemple précédent) et du platine en solution à raison de 0,5 g/l pour Au et 2,5 g/l pour Pt. La solution contenait du chlorure de sodium en sel de fond (3 moles) et était acidifiée à l'aide d'acide chlorhydrique (1 N). On a obtenu les résultats groupés dans le tableau 2.

Tableau 2

| Ex. | Extractant | Au | | Pt | |
|---|---|---|---|---|---|
| | | Phase organique g/l | Phase aqueuse g/l | Phase organique g/l | Phase aqueuse g/l |
| 14 | Butex ® [1] | 0,465 | 0,035 | 0 | 2,5 |
| 15 | Composé de l'exemple 5[2] | 0,500 | 0,000 | 0,05 | 2,45 |
| 16 | Composé de l'exemple 5[3] | 0,370 | 0,130 | 0 | 2,5 |
| 17 | Composé de l'exemple 5[4] | 0,390 | 0,110 | 0 | 2,5 |

[1]) le Butex ®, de formule Bu 0 $CH_2CH_2OCH_2OBu$, est un extractant connu Il était employé ici à l'état pur.
[2]) en solution à 445 g/l (0,5 mole) dans le chloroforme.
[3]) en solution à 44,5 g/l (0,05 mole) dans le chloroforme.
[4]) en solution à 200 g/l (0,4 mole) dans le chloroforme.

On voit que les composés selon l'invention présentent les mêmes qualités (sélectivité, capacité) que le Butex mais peuvent s'utiliser dilués alors que le Butex® doit être utilisé pur.

Exemple 18

Extraction de l'or en simulateur

Le composé de l'exemple 4 en solution 0,2 mole dans le chloroforme a été utilisé pour extraire l'or contenu à état dissous à raison de 1000 ppm dans une phase aqueuse renfermant du chlorure de sodium comme sel de fond (2 moles) et acidifiée à l'acide chlorhydrique (0,443 N du départ).

Ces phases ont été soumises aux conditions de l'AKUFVE 110 (mixage intime, centrifugation des phases, extraction en continu) pendant 4 heures en faisant varier la concentration en $H^+$.

On a mesuré le rapport $D = \frac{\text{concentration d'Au phase organique}}{\text{concentration d'Au phase aqueuse}}$ en fonction de la concentration en $H^+$. Les résultats sont rapportés au tableau 3.

Tableau 3

| | | | | | | | | | | |
|---|---|---|---|---|---|---|---|---|---|---|
| D | 0,70 | 0,72 | 0,73 | 0,75 | 0,77 | 0,82 | 0,93 | 1,09 | 1,27 | 1,65 |
| ($H^+$) (M : 1) | 0,5 | 0,75 | 1,0 | 1,25 | 1,50 | 1,75 | 2,00 | 2,25 | 2,5 | 2,75 |

Au terme de l'expérience on n'a constaté aucune altération de l'agent extractant.

L'or métallique a été réobtenu par strippage aqueux à l'aide de l'acide oxalique selon la méthode décrite par B. F. Rimmer, Chemistry and Industry, pages 63–66, du 19 janvier 1974. Le schéma réactionnel est:

$$3\,(COOH)_2 + 2H\,AuCl_4 \rightarrow 2\,Au + 6\,CO_2 + 8\,HCl$$

Par ailleurs, dans les mêmes conditions, on a pu établir que Rh (III) n'était pas extrait par le di(carbonyl dioxy-2,3 propyle carbonate) de tétraéthylèneglycol utilisé.

Il est enfin à noter que l'extraction de l'or peut être également réalisée à l'aide de ce composé, en présence d'anions nitrates en plus des anions chlorures.

Exemples 19 à 25

Séparation Uranium, Molybdène, strontium

On a tenté de résoudre la séparation du Mo (V) d'une solution aqueuse contenant également U (VI) et $Sr^{2+}$.

On a utilisé le di(carbonyl dioxy-2,3 propyle carbonate) de tétraéthylène glycol comme agent extractant en solution dans le dichloroéthane, la phase aqueuse renfermant en outre du thiocyanate d'ammonium (2 M).

On a d'abord essayé d'extraire le molybdène seul en en faisant varier la concentration, ainsi que le pH et la concentration d'acide ascorbique, dans la phase aqueuse (cf. tableau 4).

Tableau 4

| Exemple | Mo ppm | $(H^+)$ | acide ascorbique |
|---|---|---|---|
| 19 | 500 | $10^{-1}$ | 25 g/l |
| 20 | 250 | $5 \cdot 10^{-2}$ | 12,50 g/l |
| 21 | 100 | $2 \cdot 10^{-2}$ | 5 g/l |

Dans ces conditions (l'acide ascorbique est destiné à réduire Mo (VI) en Mo (V)) on a obtenu les extractions rapportées au tableau

Tableau 5

| | Concentration extractant (g/l) | | | | | | |
|---|---|---|---|---|---|---|---|
| | 0 | 20 | 30 | 60 | 100 | 150 | 200 |
| % Mo extrait exemple 19 | 0 | 12 | 16 | 18 | 21 | 32 | 40 |
| % Mo extrait exemple 20 | 0 | 5,2 | 5,6 | 9,7 | 15,4 | 32,6 | 56,2 |
| % Mo extrait exemple 21 | 0 | 4,7 | 8,4 | 10,3 | 19,2 | 42,5 | 54,7 |

La concentration en $H^+$ etant ensuite fixée à $2 \cdot 10^{-2}$ M et celle en thiocyanate à 2 M, on a mesuré respectivement aux exemples 22 à 25 le pourcentage extrait de Mo, Sr, U (VI) et U (VI) á nouveau, à partir de solution aqueuse contenant respectivement 100 ppm de Mo et 100 ppm d'U (ex. 22), 100 ppm de Sr (ex. 23), 100 ppm d'U (ex. 24) et 100 ppm de Mo et 100 ppm d'U (ex. 25). Les résultats obtenus en fonction de la concentration du même extractant qu'aux exemples 19 à 21 dans le dichloroéthane, sont rapportés au tableau 6:

Tableau 6

| | Concentration extractant (g/l) | | | | | | |
|---|---|---|---|---|---|---|---|
| | 0 | 20 | 30 | 60 | 100 | 150 | 200 |
| % Mo extrait (exemple 22) | 0 | 5,6 | 4,7 | 12,6 | 30,4 | 43,5 | 40,2 |
| % Sr extrait (exemple 23) | 1 | 1 | 1 | 1 | 1 | 1 | 1 |
| % U (VI) extrait (exemple 24) | 0 | 0 | 0 | 0 | 1 | 2 | |
| % U (VI) extrait (exemple 25) | 0 | 0 | 0 | 0 | 1 | 3,7 | |

Ces résultats montrent la possibilité d'extraire Mo, puis U (VI) sans extraire Sr, d'une manière très satisfaisante.

### Exemple 26

### Extraction sélective de Co en présence de Ni

On a utilisé des solutions de di(carbonyl dioxy-2,3 propyle carbonate) de tétraéthylène glycol dans le chloroforme en vue d'extraire le cobalt contenu composé raison de 100 ppm dans une solution aqueuse à pH = 2 contenant en outre une solution molaire de thiocyanate de potassium et 200 ppm de $Ni^{++}$.

Les résultats obtenus en fonction de la concentration en M/l d'extractant sont rapportés dans le tableau 7.

Tableau 7

| | Concentration extract. (M/l) | | | | | |
|---|---|---|---|---|---|---|
| | 0,127 | 0,204 | 0,233 | 0,271 | 0,311 | 0,498 |
| % Co extrait | 0 | 7 | 13 | 25 | 39 | 90 |
| % Ni extrait | 0 | 0 | 0 | 0 | 0 | 0 |

Ces résultats montrent la possibilité de séparer le cobalt du nickel auquel il est fréquemment associé à l'aide des composés selon l'invention.

### Exemple 27

### Comparaison avec des produits commerciaux

On a comparé le composé selon l'invention de formule:

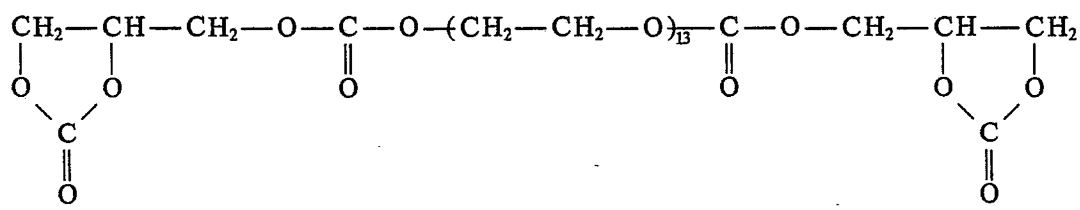

de masse 890, avec un polyéthylène glycol

$$HO-(CH_2CH_2O)_{20}-H$$

(masse: 1000) et avec le Brij 35® de formule

$$C_{12}H_{25}O-(CH_2CH_2O)_n-H$$

(où n = 23 et de masse 1200).

A cette fin on a réalisé des solutions à 10 g/l de ces extractants dans le dichloroéthane, la phase aqueuse étant deux fois molaire en $NH_4SCN$ et ayant un pH égal à 1, ajusté avec de l'acide chlorhydrique. On a mesuré l'extraction rélaisée par mélange des phases organiques et aqueuses ainsi obtenues, la phase aqueuse contenant 100 ppm des divers métaux testés (10 ppm dans le cas de Hg). Les pourcentages d'extraction sont indiqués au tableau 8:

Tableau 8

| | Extractant | | | | | | | | |
|---|---|---|---|---|---|---|---|---|---|
| | Zn (II) | Mo (V) | Co (II) | Fe (III) | Hg (II) | V (IV) | Cu (I) | Pu (II) | Cd (II) |
| Composé B | 99 | 98 | 95,2 | 80 | 26 | 20 | 31,2 | 2,4 | 4,8 |
| P E G 1000 | 99 | 91 | 87 | 34 | 11 | 8 | 7 | 2 | 3 |
| Brij 35 • | 100 | 99 | 99 | 92 | 56 | 75 | 38 | 12 | – |

On voit que le composé selon l'invention a un pouvoir extractant comparable à celui de produits de commerce alors qu'il possède une masse et un nombre de ponts éther notablement plus faible. Ceci montre donc l'effet extrêment favorable de la conjonction du groupe carbonate cyclique avec une chaine polyéther.

On a également noté que le composé B est capable d'extraire, dans les conditions précédentes, faiblement mais très sélectivement, le Praséodyme (4%).

Enfin, le composé B extrayant Zn mais non Ni, il permet de réaliser la séparation de ces métaux sans avoir recours à l'utilisation d'acide phosphonique à l'instar du procédé décrit dans le brevet français 2 367 832.

Exemples 28 à 37

On a voulu rechercher par quel type de mécanisme les composés selon l'invention intervenaient en tant qu'extractants. On sait par exemple que des composés du type polyéther tel que le Butex mentionné plus haut extraient les valeurs métalliques par solvatation liée à la protonation dudit extractant en milieu très acide:

$$M^{n+} + (n+1)X^- + H^+ + \overline{EXTR} \rightleftharpoons \overline{EXTR^+H\,MX^-_{n+1}}$$

(où M est le métal, n sa valence, $X^-$ l'anion du sel de fond et EXTR l'extractant). Dans un tel mécanisme le pH a évidemment une grande influence, et doit être généralement inférieur ou égal à zéro.

Si cette dernière condition parait lourde on peut extraire par un mécanisme de solvatation proprement dite:

$$M^{n+} + nX^- + \overline{EXTR} \rightleftharpoons \overline{X_3M\,EXTR}$$

Dans ce cas, l'extractant ne complexe que partiellement le métal et son activité dépend notablement du sel de fond, ce qui constitue une autre sujétion.

Au contraire, avec les composés selon l'invention, c'est la nature du cation $M_s^+$ du sel de fond qui revêt une grande importance, le pH pouvant être positif, par exemple, compris entre 1 et 5, sans inconvénients; le schéma de la réaction d'extraction pouvant être:

$$M_s^+ + M^{3+} + 4X^- + n\,\overline{EXTR} \rightleftharpoons M_s(EXTR)_n + MX_4^-$$

Pour démontrer que les composés selon l'invention peuvent notamment extraire les valeurs métalliques par ce mécanisme dit d'extraction par complexation d'alcalin on a d'abord réalisé des solutions aqueuses de thiocyanate de potassium (M) en milieu de pH 2; la concentration de l'extractant variant dans du dichloroéthane (tableau 9).

Tableau 9

| Extractants | | | | | | | |
|---|---|---|---|---|---|---|---|
| Butex (Ex. 28) | | Debcy (Ex. 29)[1] | | Tebcy (Ex. 30)[2] | | Qebcy (Ex. 31)[3] | |
| [Extr] M/l | $[K^+]$ $10^{-3}$ M/l | [Extr] M/l | $[K^+]$ $10^{-3}$ M/l | [Extr] M/l | $[K^+]$ $10^{-3}$ M/l | [Extr] M/l | $[K^+]$ $10^{-3}$ M/l |
| | | | | | | 0,10 | 0,4 |
| | | | | | | 0,20 | 1,0 |
| | | 0,35 | 0,3 | 0,30 | 0,6 | 0,30 | 2,2 |
| 0,45 | 0,35 | 0,40 | 0,4 | 0,40 | 1,0 | 0,40 | 3,7 |
| 0,50 | 0,40 | 0,50 | 0,7 | 0,50 | 1,9 | 0,50 | 6,8 |
| 0,60 | 0,40 | 0,60 | 1,0 | 0,60 | 2,3 | 0,55 | 8,5 |
| 0,70 | 0,45 | 0,70 | 1,4 | 0,70 | 3,7 | | |
| 0,80 | 0,50 | 0,80 | 2,0 | 0,80 | 5,1 | | |
| 0,90 | 0,55 | 0,90 | 2,6 | | | | |
| 1,00 | 0,6 | 1,00 | 3,7 | | | | |

[1]) Carbonate de bis(carbonyl dioxy-2,3 propyle) et de diéthylène glycol.
[2]) Carbonate de bis(carbonyl dioxy-2,3 t=de propyle) et de triéthylène glycol.
[3]) Carbonate de bis (carbonyl dioxy-2,3 propyle) et de tétraéthylène glycol.

Il résulte de ce tableau que le BUTEX® n'extrait pratiquement pas le potassium par rapport au DEBCY qui comprend le même nombre de ponts éther et, à fortiori, par rapport au TEBCY et QEBCY.

Cette constatation étant faite on a étudié l'influence de la nature du cation alcalin associé sur l'extraction de 100 ppm de $Co^{2+}$ ajoutés à la solution aqueuse précédente (molaire en sel de fond, pH=2). Les tableaux 10 et 11 rapportent les résultats obtenus respectivement avec le carbonate de bis(carbonyl dioxy-2,3 propyle) et de triéthylène glycol et le carbonate de bis(carbonyl dioxy-2,3 propyle) et de tétraéthylène glycol.

Tableau 10

| Ex | | Extractant (M/l) | | | | | |
|---|---|---|---|---|---|---|---|
| | | 0,12 | 0,18 | 0,3 | 0,36 | 0,48 | 0,60 |
| 32 | % $Co^{2+}$ extrait NaSCN | 0 | 0 | 0 | 0 | 1 | 10 |
| 33 | % $Co^{2+}$ extrait $NH_4SCN$ | 0 | 0 | 2 | 6 | 15 | 30 |
| 34 | % $Co^{2+}$ extrait KSCN | 2 | 4 | 14 | 25 | 55 | 77 |

Tableau 11

| Ex | | Extractant (M/l) | | | | | |
|---|---|---|---|---|---|---|---|
| | | 0,12 | 0,18 | 0,3 | 0,36 | 0,48 | 0,60 |
| 35 | % $Co^{2+}$ extrait NaSCN | 0 | 0 | 4 | 9 | 23 | 34 |
| 36 | % $Co^{2+}$ extrait $NH_4SCN$ | 5 | 11 | 30 | 49 | 77 | 92 |
| 37 | % $Co^{2+}$ extrait KSCN | 17 | 46 | 85 | 92 | 96 | 99 |

On voit l'influence extrêmement favorable de la présence du cation $K^+$ dans la phase aqueuse vis à vis de l'extraction de $Co^{2+}$. Cet effet est ressenti quel que soit l'anion associé à $K^+$ dans le sel de fond de sorte que l'extraction peut être effectuée avec d'excellents résultats, à un pH très modéré avec un sel de fond bon marché tel que KCl. Ceci est particulièrement intéressant pour le traitement des eaux résiduaires faiblement acides où les valeurs métalliques sont fortement diluées.

Exemple 38

Synthèse du carbonate de phényle et de carbonyl dioxy-2,3 propyle

Dans un réacteur de 500 $cm^3$, on place 51,7 g (0,55 mole) de phénol, 41,5 g (0,52 mole) de pyridine et 300 $cm^3$ de chloroforme. A cette solution maintenue à 0°C on additionne 90,3 g (0,5 mole) de chloroformiate de carbonyl dioxy-2,3 propyle dilué dans 50 $cm^3$ de chloroforme. On agite 2 heures à température ambiante puis on lave le mélange réactionnel par 200 $cm^3$ d'eau acidulée, 200 $cm^3$ d'eau pure, 200 $cm^3$ d'eau carbonatée et enfin 200 $cm^3$ d'eau pure. La phase organique après séchage est évaporée sous pression réduite. On obtient ainsi 80,7 g (rendement: 71,5%) de carbonate de phényle et de carbonyl dioxy-2,3 propyle fondant à 94° C.

– Spectre Infra Rouge: $>C=O$ linéaire 1750 $cm^{-1}$

$>C=O$ cyclique 1800 $cm^{-1}$

$>C=C<$ benzénique 1590 $cm^{-1}$

– Spectre RMN:

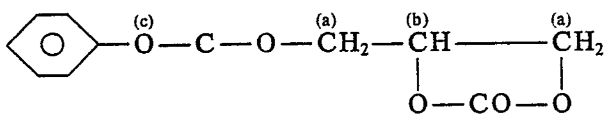

(a) massif centré à 4,63 ppm (4H)
(b) massif centré à 5,25 ppm (1H)
(c) massif centré à 7,35 ppm (5H).

Exemple 39

Carbonate de méthyle et de carbonyl dioxy-2,3 propyle

Dans un réacteur de 500 $cm^3$ on place 21,1 g (0,66 mole) de méthanol, 49,8 g (0,68 mole) de pyridine et 300 $cm^3$ de chloroforme. La température à 0° C, on additionne 108,2 g (0,60 mole) de chloroformiate de carbonyl dioxy-2,3 propyle dilué dans 50 $cm^3$ de chloroforme. Après deux heures d'agitation à température ambiante le mélange réactionnel est lavé à l'eau acidulée puis à l'eau pure.

Après séchage, la phase organique est concentrée sous pression réduite. On obtient ainsi 42,8 g (rendement 40%) de carbonate de méthyle et de carbonyl dioxy-2,3 propyle fondant à 90° C.

– Spectre Infra Rouge: $>C=O$ cyclique: 1800 $cm^{-1}$

$>C=O$ linéaire: 1740 $cm^{-1}$

– Spectre RMN:

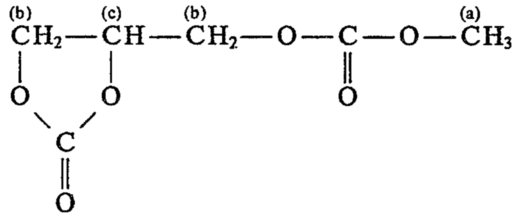

(a) singulet à 3,75 ppm (3H)
(b) massif centré à 4,45 ppm (4H)
(c) massif centré à 5,10 ppm (1H).

**Revendications**

1. Carbonates porteurs de groupes carbonates cycliques de formule générale:

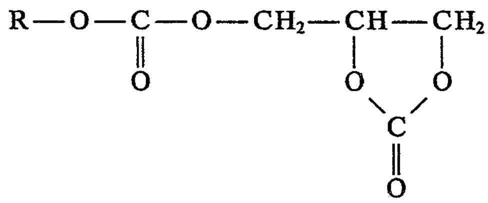

où R est un groupe alkyle linéaire ou ramifié comportant de 1 à 20 atomes de carbone, éventuellement substitué par un à trois groupe

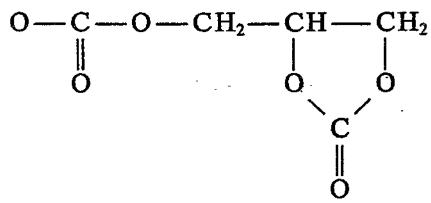

– un groupe alicyclique comportant de 4 à 20 atomes de carbone, ou un groupe aralkyle comportant de 7 à 20 atomes de carbone, éventuellement substitués par un à trois groupes

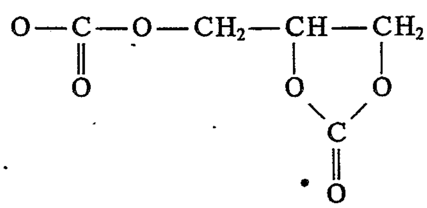

– un groupe carboxylate –COOR″ où R″ comporte de 1 à 19 atomes de carbone, éventuellement substitué par un groupe

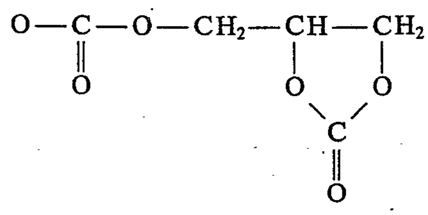

– un groupe méthacryloyloxyéthyle

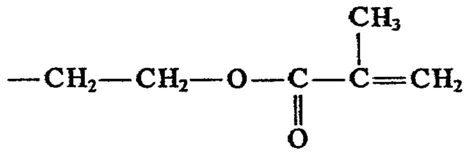

– un polyoxyéthylène de formule:

$$R'-\!\!-(O-CH_2-CH_2)_n-$$

où n est compris entre 1 et 40 où R' est un groupe hydrocarbyle comprenant de 1 à 10 atomes de carbone et porte éventuellement une ou deux chaines

$$-(O-CH_2-CH_2)_{n'}-,$$

où n' est compris entre 1 et 40, terminées par un groupe

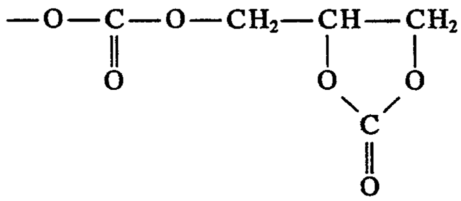

– un polyoxypropylène de formule

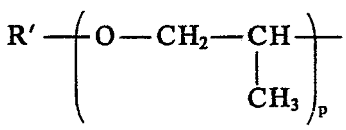

où p est compris entre 1 et 40 et où R' est un groupe hydrocarbyle comprenant de 1 à 10 atomes de carbone et porte éventuellement une ou deux chaines

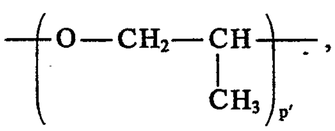

où p' est compris entre 1 et 40, terminées par un groupe

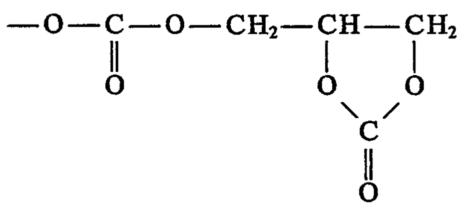

– un groupe aryle comportant de 6 à 20 atomes de carbone,
– un polyester de formule

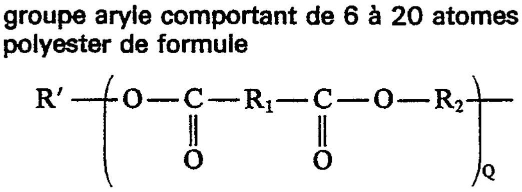

où Q est compris entre 1 et 20, où $R_1$ et $R_2$ sont identiques ou différents et sont une chaine polyméthylène ayant de 1 à 8 atomes de carbone ou une chaine polyéther ayant de 1 à 8 atomes de carbone, et où R' est un groupe hydrocarbyle comprenant de 1 à 10 atomes de carbone, ou bien,

– un polycarbonate aliphatique

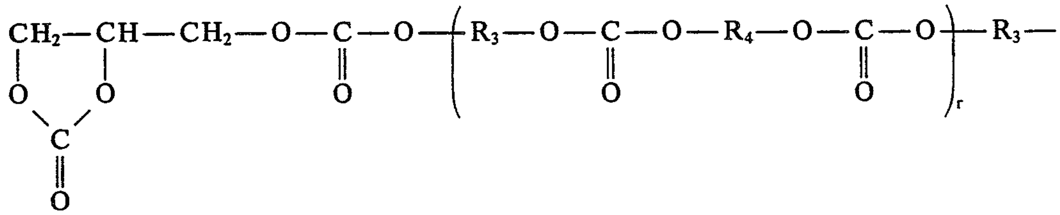

où r est compris entre 1 et 20 et où $R_3$ et $R_4$ sont identiques ou différents ou tantôt identiques, tantôt différents et sont des groupes polyméthylène comportant de 2 à 8 atomes de carbone ou des groupes polyoxyéthylène

$$-(CH_2-CH_2-O)_t-CH_2-CH_2-$$

où t est égal à 1, 2, 3, 4 ou 5.

2. Procédé de synthèse des carbonates selon la revendication 1, caractérisé en ce qu'on fait agir le chloroformiate de carbonyle dioxy-2,3 propyle sur un alcool de formule générale R OH où R a l'une des significations donées à la revendication 1, des groupes OH remplaçant les groupes

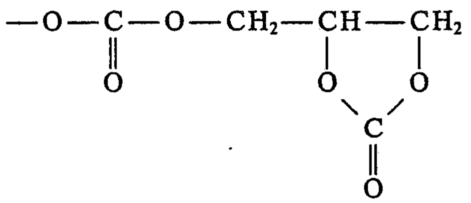

éventuels, à une température comprise entre −10 et +20° C et en présence d'un accepteur d'acide.

3. Procédé selon la revendication 2, caractérisé en ce que l'accepteur d'acide est une base minérale choisie dans le groupe constitué par la soude, la potasse, le carbonate de sodium, le carbonate de potassium et le bicarbonate de sodium.

4. Procédé selon la revendication 2, caractérisé en ce que l'accepteur d'acide est une base organique choisie dans le groupe constitué par la pyridine et les amines tertiaires aliphatiques.

5. Procédé selon l'une quelconque des revendications 2 à 4 caractérisé en ce qu'on effectue la réaction en outre dans un solvant choisi dans le groupe constitué par le produit lui-même, les hydrocarbures aliphatiques, les hydrocarbures aromatiques, les hydrocarbures halogénés, les éthers, les cétones et les esters.

6. Procédé selon l'une quelconque des revendications 2 à 5 caractérisé en ce qu'on fait réagir au moins une mole de chloroformiate de carbonyl dioxy-2,3 propyle par mole d'hydroxyle protée par alcool, en présence d'une quantité équimoléculaire d'accepteur d'acide.

7. Procédé selon la revendication 6 caractérisé en ce que l'excès de chloroformiate est compris entre 5 et 15% molaire.

8. Procédé de séparation de valeurs métalliques à partir d'une solution aqueuse les contenant par extraction liquide-liquide caractérisé en ce que (1) on met lesdites solutions aqueuses en contact avec une phase organique comprenant un composér selon la revendication 1 de formule générale (A) éventuellement en solution dans un solvant organique sensiblement non miscible à l'eau, si bien qu'une partie au moins desdites valerus métalliques est extraite dans la phase organique (2) on sépare la phase organique chargée contenant les valeurs métalliques extraites de la solution aqueuse sous forme d'un complexe avec le composé de formule générale (A) et (3) on récupère les valeurs métalliques de la phase organique par mise en contact de cette dernière avec un milieu de strippage aqueux.

9. Procédé selon la revendication 8, caractérisé en ce que les valeurs métalliques traitées sont choisies dans le groupe constitué par Zn (II), Cd (II), Hg (II), Fe (III), Co (II), Cu (I), Cu (II), Rh (III), Pd (II), Pt (II), Mo (V), V (IV), Pb (II), Ag (I), Au (III), Ga (III), Na (I), K (I), Hg (I), U (IV), U (VI) et les lanthanides aux degrés d'oxydation (II), (III) ou (IV).

10. Procédé selon l'une quelconque des revendications 8 ou 9, caractérisé en ce que le composé extractant est choisi dans le groupe constitué par le carbonate de carbonyl dioxy-2,3 propyle et de méthyle, de n-propyle, de n-butyle, de n-pentyle, de n-hexyle, de n-heptyle, de n-octyle et leurs homologues linéaires supérieurs jusqu'à octa décyle, le carbonate carbonyl dioxy-2,3 propyle et de benzyle, le carbonate de carbonyl dioxy-2,3 propyle et de phényle, le carbonate tri(carbonyl dioxy-2,3 propyle) et triméthy ol propane, les carbonates de bis(carbonyl dioxy-2,3 propyle) et polycarbonate de diéthylène glycol de masse inférieure à 3000, les carbonates de bis(carbonyl dioxy-2,3 propyle) et de polycarbonate de butane diol de masse inférieure à 2000, les carbonates de bis(carbonyl dioxy-2,3

propyle) et de polycarbonates séquencés ou non d'hexane diol et de butane diol, les polyoxyéthylènes de formule

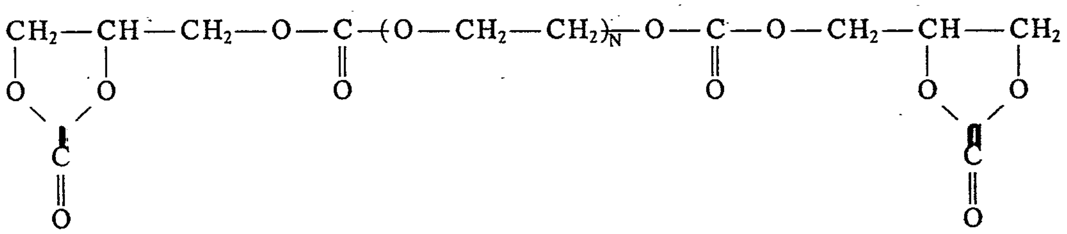

où N est égal ou supérieur à 2 et dont la masse molaire ne dépasse pas, de préférence, 5000, les carbonates de bis(carbonyl dioxy-2,3 propyle) et de diol et les carbonates de tri(carbonyl dioxy-2,3 propyle) et de triol, obtenus par condensation d'oxyde de propylène sur des diols ou des triols aliphatiques comprenant de 2 à 8 atomes de carbone, et de masse globale inférieure de préférence à 3000.

11. Procédé selon l'une quelconque des revendications 8 à 10, caractérisé en ce que le pH de la phase aqueuse chargée des valeurs métalliques à extraire et le pH du milieu de strippage est compris entre — 1 et 12, de préférence entre 0 et 10.

**Patentansprüche**

1. Carbonate mit cyclischen Carbonatgruppen der allgemeinen Formel

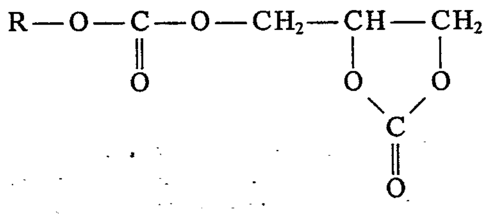

in der R bedeutet:

- geradkettiges oder verzweigtes $C_1$- bis $C_{20}$-Alkyl, das gegebenenfalls mit ein bis drei Gruppen

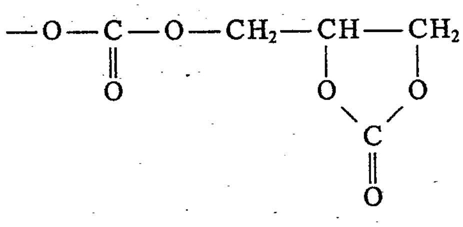

substituiert ist,

- eine alicyclische Gruppe mit 4 bis 20 C-Atomen oder eine Aralkylgruppe mit 7 bis 20 C-Atomen, die beide gegebenenfalls mit ein bis drei Gruppen der Formel

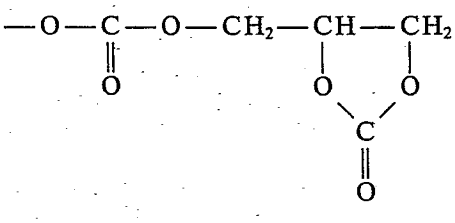

substituiert sind,

– eine Carboxylatgruppe – COOR" wobei R" 1 bis 19 C-Atome aufweist und gegebenenfalls mit einer Gruppe der Formel

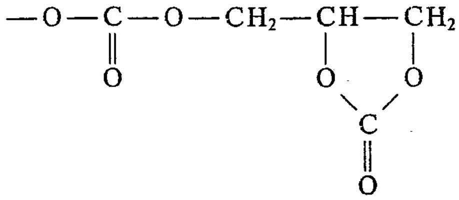

substituiert ist,

– Methacryloyloxyethyl der Formel

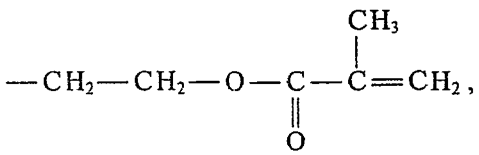

– eine Polyoxyethylengruppe der Formel

$$R'-(O-CH_2-CH_2)_n-$$

in der n eine Zahl von 1 bis 40 und R' eine Kohlenwasserstoffgruppe mit 1 bis 10 C-Atomen darstellt, die gegebenenfalls eine oder zwei Ketten der Formel

$$-(O-CH_2-CH_2)_{n'}-,$$

trägt, in der n' eine Zahl von 1 bis 40 bedeutet, die eine Endgruppe der Formel

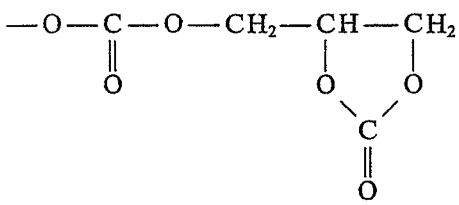

aufweisen,

– eine Polyoxypropylengruppe der Formel

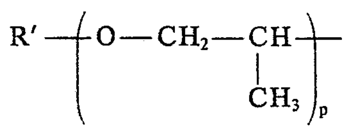

in der p eine Zahl von 1 bis 40 und R' eine Kohlenwasserstoffgruppe mit 1 bis 10 C-Atomen, die gegebenenfalls mit einer oder zwei Ketten der Formel

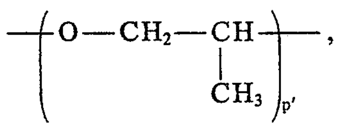

substituiert ist, bedeuten, wobei p' eine Zahl von 1 bis 40 darstellt, die eine Endgruppe der Formel

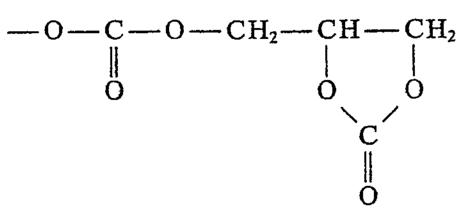

aufweisen,

– eine Arylgruppe mit 6 bis 20 C-Atomen,

– eine Polyestergruppe der Formel

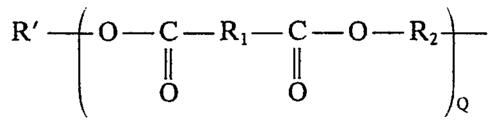

in der Q eine Zahl von 1 bis 20 und $R_1$ und $R_2$, die gleich oder verschieden sein können, eine Polymethylenkette mit 1 bis 8 C-Atomen oder eine Polyetherkette mit 1 bis 8 C-Atomen und R' eine Kohlenwasserstoffgruppe mit 1 bis10 C-Atomen bedeuten,
oder

– eine aliphatische Polycarbonatgruppe der Formel

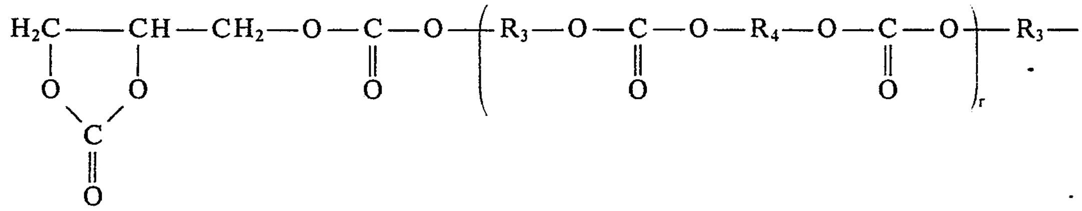

in der r eine Zahl von 1 bis 20 und $R_3$ und $R_4$, die gleich oder verschieden oder zum Teil gleich und zum Teil verschieden sein können, Polymethylengruppen mit 2 bis 8 C-Atomen oder Polyoxyethylengruppen der Formel

$$-(CH_2-CH_2-O)_t-CH_2-CH_2-$$

bedeuten, wobei t 1, 2, 3, 4 oder 5 ist.

2. Verfahren zur Herstellung der Carbonate nach Anspruch 1, gekennzeichnet durch Umsetzung von 2.3-Dioxycarbonylpropyl-chlorformiat mit einem Alkohol der allgemeinen Formel R – OH, in der R dasselbe wie in Anspruch 1 bedeutet, gegebenenfalls unter Ersatz der Gruppen der Formel

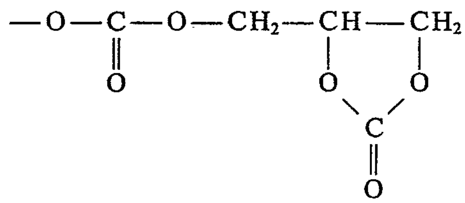

durch OH-Gruppen bei einer Temperatur von –10 bis +20° C und in Gegenwart eines Säureakzeptors.

3. Verfahren nach Anspruch 2, gekennzeichnet durch Verwendung einer unter Natriumhydroxid, Kaliumhydroxid, Natriumcarbonat, Kaliumcarbonat und Natriumhydrogencarbonat ausgewählten anorganischen Base als Säureakzeptor.

4. Verfahren nach Anspruch 2, gekennzeichnet durch Verwendung einer unter Pyridin und aliphatischen tertiären Aminen ausgewählten organischen Base als Säureakzeptor.

5. Verfahren nach einem der Ansprüche 2 bis 4, gekennzeichnet durch Durchführung der Reaktion in einem unter dem Produkt selbst, aliphatischen Kohlenwasserstoffen, aromatischen Kohlenwasserstoffen, halogenierten Kohlenwasserstoffen, Ethern, Ketonen und Estern ausgewählten Lösungsmittel.

6. Verfahren nach einem der Ansprüche 2 bis 5, gekennzeichnet durch Umsetzung von mindestens 1 mol 2.3-Dioxycarbonylpropyl-chlorformiat pro Mol Hydroxylgruppen des Alkohols in Gegenwart einer äquimolaren Menge an Säureakzeptor.

7. Verfahren nach Anspruch 6, gekennzeichnet durch Anwendung des Chlorformiats in einem Überschuß von 5 bis 15 mol-%.

8. Verfahren zur Abtrennung von Metallionen aus metallionenhaltigen wäßrigen Lösungen durch Flüssig-Flüssig-Extraktion, gekennzeichnet durch

(1) Inkontaktbringen der wäßrigen Lösungen mit einer organischen Phase, die eine Verbindung nach Anspruch 1 der allgemeinen Formel (A), gegebenenfalls in Lösung in einem im wesentlichen nicht mit Wasser mischbaren organischen Lösungsmittel, so daß mindestens ein Teil der Metallionen in die organische Phase extrahiert wird,
(2) Abtrennung der beladenen organischen Phase, die die extrahierten Metallionen in Form eines Komplexes mit der Verbindung der allgemeinen Formel (A) enthält, von der wäßrigen Lösung und
(3) Gewinnung der Metallionen aus der organischen Phase durch Inkontaktbringen der organischen Phase mit einem wäßrigen Strippermedium.

9. Verfahren nach Anspruch 8, dadurch gekennzeichnet, daß Zn(II), Cd(II), Hg(II), Fe(III), Co(II), Cu(I), Cu(II), Rh(III), Pd(II), Pt(II), Mo(V), V(IV), Pb(II), Ag(I), Au(III), Ga(III), Na(I), K(I), Hg(I), U(IV), U(VI) und Lanthaniden in der Oxidationsstufe (II), (III) und (IV) abgetrennt werden.

10. Verfahren nach Anspruch 8 oder 9, dadurch gekennzeichnet, daß das Extraktionsmittel ausgewählt wird unter 2.3-Dioxycarbonylpropyl-methylcarbonat, 2.3-Dioxycarbonylpropyl-n-propylcarbonat, 2.3-Dioxycarbonylpropyl-n-propylcarbonat, 2.3-Dioxycarbonylpropyl-n-butylcarbonat, 2.3-Dioxycarbonylpropyl-n-pentylcarbonat, 2.3-Dioxycarbonylpropyl-n-hexylcarbonat, 2.3-Dioxycarbonylpropyl-n-heptylcarbonat, 2.3-Dioxycarbonylpropyl-n-octylcarbonat und den entsprechenden 2.3-Dioxycarbonylpropylcarbonaten mit den entsprechenden höheren homologen linearen Gruppen bis Octadecyl, 2.3-Dioxycarbonylpropyl-benzylcarbonat, 2.3-Dioxycarbonylpropyl-phenylcarbonat, Tris(2.3-dioxycarbonylpropyl)-trimethylolpropancarbonat, Bis(2.3-dioxycarbonylpropyl)-carbonaten mit Diethylenpolycarbonatgruppen mit einem Molekulargewicht unter 3000, Bis(2.3-dioxycarbonylpropyl)-carbonaten mit Butandiolpolycarbonaten mit einem Molekulargewicht unter 2000, Bis(2.3-dioxycarbonylpropyl)-carbonaten von sequentiellen oder nichtsequentiellen Polycarbonaten von Hexandiol und Butandiol, Polyoxyethylenen der Formel

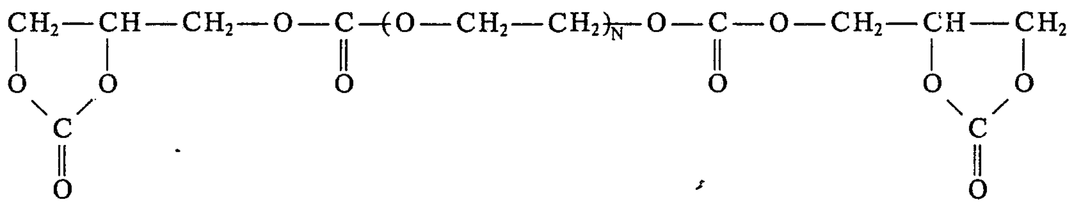

in der $N \geq 2$ ist, mit einem Molekulargewicht von vorzugsweise unter 5000, Bis(2.3-dioxycarbonylpropyl)-carbonaten mit Diolen und Tris(2.3-dioxycarbonylpropyl)-carbonaten von Triolen, die durch Kondensation von Propylenoxid mit aliphatischen Diolen oder Triolen mit 2 bis 8 C-Atomen erhalten sind und ein Molekulargewicht von vorzugsweise unter 3000 aufweisen.

11. Verfahren nach einem der Ansprüche 8 bis 10, dadurch gekennzeichnet, daß der pH-Wert der mit den zu extrahierenden Metallionen beladenen wäßrigen Phase und der pH-Wert des Strippermediums zwischen 1 und 12 und vorzugsweise zwischen 0 und 10 liegen.

## Claims

1. Carbonates carrying cyclic carbonate groups, of the general formula:

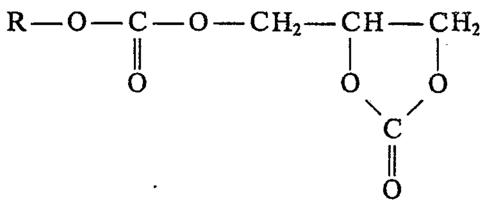

in which R is a linear or branched alkyl group which contains from 1 to 20 carbon atoms, optionally substituted by one to three groups

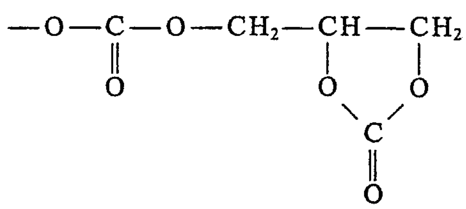

- an alicyclic group which contains 4 to 20 carbon atoms, or an aralkyl group which contains 7 to 20 carbon atoms, optionally substituted by one to three groups

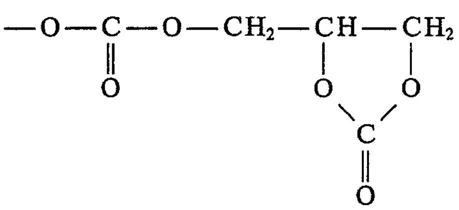

– a carboxylate group COOR″ in which R″ contains from 1 to 19 caron atoms, optionally substituted by a group

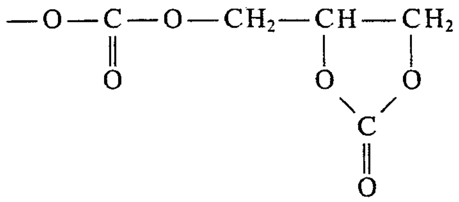

– a methacryloyloxyethyl group

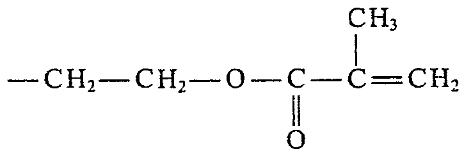

– a polyoxyethylene of the formula

$$R'-(O-CH_2-CH_2)_n-$$

in which n is between 1 and 40 and R′ is a hydrocarbyle group containing from 1 to 10 carbon atoms and optionally carries one or two chains

$$-(O-CH_2-CH_2)_{n'}-,$$

in which n′ is between 1 and 40, which chains are terminated by a group

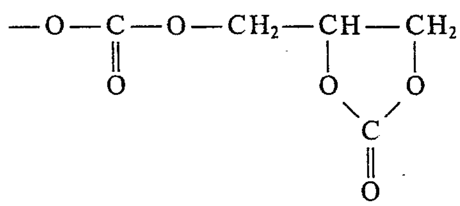

– a polyoxypropylene of the formula

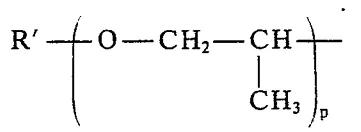

in which p is between 1 and 40 and R′ is a hydrocarbyle group containing from 1 to 10 carbon atoms and optionally carries one or two chains

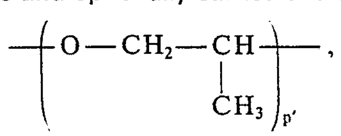

in which p′ is between 1 and 40, which chains are terminated by a group

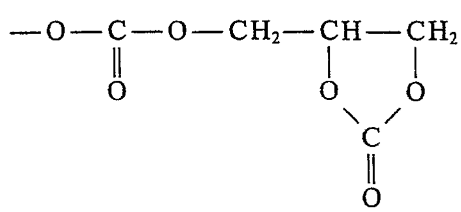

– an aryl containing from 6 to 20 carbon atoms,

– a polyester of the formula:

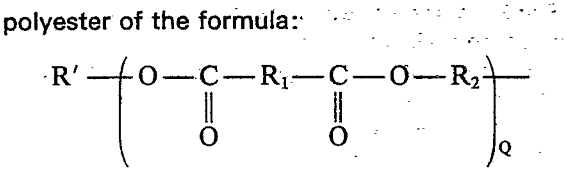

in which Q is between 1 and 20, $R_1$ and $R_2$ are identical or different and are a polymethylene chain having from 1 to 8 carbon atoms or a polyether chain having from 1 to 8 carbon atoms, and R' is a hydrocarbyle group containing from 1 to 10 carbon atoms, or alternatively

– an aliphatic polycarbonate

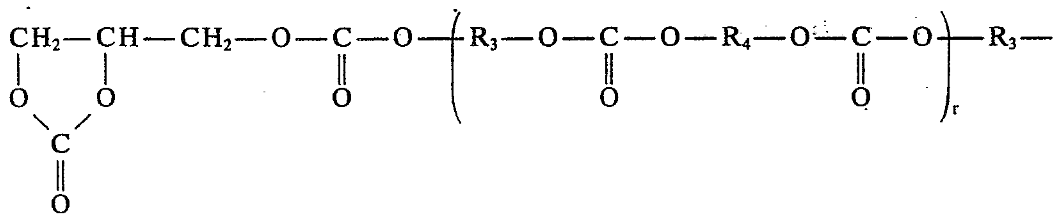

in which r is between 1 and 20 and $R_3$ and $R_4$ are identical or different, or in some cases identical and in other cases different, and are polymethylene groups containing from 2 to 8 carbon atoms or polyoxyethylene groups

$$-(CH_2-CH_2-O)_t-CH_2-CH_2-$$

in which t is equal to 1, 2, 3, 4 or 5.

2. Process for synthesising the carbonates according to Claim 1, characterised in that 2,3-dioxycarbonylpropyl chloroformate is reacted with an alcohol of the general formula ROH, in which R has one of the meanings given in Claim 1, OH groups replacing the groups

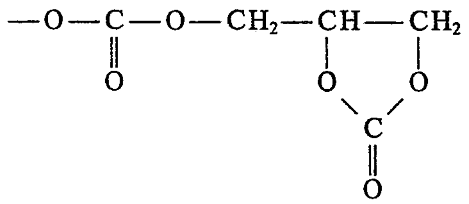

where appropriate, at a temperature between −10 and +20°C and in the presence of an acid acceptor.

3. Process according to Claim 2, characterised in that the acid acceptor is an inorganic base chosen from the group consisting of sodium hydroxide, potassium hydroxide, sodium carbonate, potassium carbonate and sodium bicarbonate.

4. Process according to Claim 2, characterised in that the acid acceptor is an organic base chosen from the group consisting of pyridine and aliphatic tertiary amines.

5. Process according to any one of Claims 2 to 4, characterised further in that the reaction is carried out in a solvent chosen from the group consisting of the product itself, aliphatic hydrocarbons, aromatic hydrocarbons, halogenated hydrocarbons, ethers, ketones and esters.

6. Process according to any one of Claims 2 to 5, characterised in that at least one mol of 2,3-dioxycarbonylpropyl chloroformate is reacted per mol of hydroxyl carried by the alcohol, in the presence of an equimolecular amount of acid acceptor.

7. Process according to Claim 6, characterised in that the excess chloroformate is between 5 and 15 mol-%.

8. Process for the separation, by liquid-liquid extraction, of metal values from an aqueous solution in which they are present, characterised in that (1) the said aqueous solutions are brought into contact with an organic phase comprising a compound according to Claim 1, of the general formula (A), optionally in solution in an essentially water-immiscible organic solvent, so that the said metal values are at least partially extracted into the organic phase, (2) the charged orgainic phase containing the metal values extracted from the aqueous solution in the form of a complex with the compound of the general formula (A) is separated off, and (3) the metal values are recovered from the organic phase by bringing the latter into contact with an aqueous stripping medium.

9. Process according to Claim 8, characterised in that the metal values treated are chosen from the group consisting of Zn(II), Cd(II), Hg(II), Fe(III), Co(II), Cu(I), Cu(II), Rh(III), Pd(II), Pt(II), Pt(II), Mo(V), V(IV), Pb(II), Ag(I), Au(III), Ga(III), Na(I), K(I), Hg(I), U(IV), U(VI) and the lanthanides in oxidation states (II), (III) or (IV).

10. Process according to either one of Claims 8 or 9, characterised in that the extraction compound is chosen from the group consisting of alkyl 2,3-dioxycarbonylpropyl carbonates in which alkyl is methyl, n-propyl, n-butyl, n-pentyl, n-hexyl, n-heptyl, n-octyl and their higher linear homologues up to octadecyl, benzyl 2,3-dioxycarbonylpropyl carbonate, phenyl 2,3-dioxycarbonylpropyl carbonate, trimethylolpropane 2,3-dioxytricarbonylpropyl carbonate, poly-[(diethylene glycol) carbonate] 2,3-dioxydicarbonylpropyl carbonates in which the polycarbonate part has a mass of less than 3,000, poly-(butanediol carbonate) 2,3-dioxydicarbonylpropyl carbonates in which the polycarbonate part has a mass of less than 2,000, poly[hexanediol/butanediol) carbonate] 2,3-dioxydicarbonylpropyl carbonates in which the polycarbonate part may or may not be in the form of a block copolymer, the polyethylenes of the formula

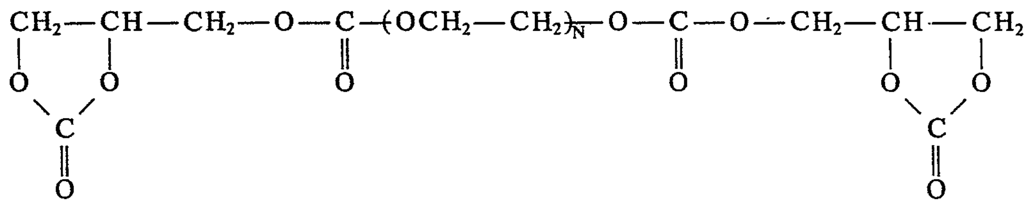

in which N is equal to or greater than 2 and of which the molecular weight preferably does not exceed 5,000, and the diol 2,3-dioxydicarbonylpropyl carbonates and triol 2,3-dioxytricarbonylpropyl carbonates obtained by condensing propylene oxide with aliphatic diols or triols containing from 2 to 8 carbon atoms, and preferably having an overall mass of less than 3,000.

11. Process according to any one of Claims 8 to 10, characterised in that the pH of the aqueous phase charged with the metal values to be extracted, and the pH of the stripping medium, is between −1 and 12, preferably between 0 and 10.